# NEUE EUROPÄISCHE PATENTSCHRIFT

(11) **EP 0 640 604 B2**
(45) Veröffentlichungstag und Bekanntmachung des Hinweises auf die Entscheidung über den Einspruch: **30.06.2004**
(45) Hinweis auf die Patenterteilung: 16.05.2001
(21) Anmeldenummer: 94810431.0
(22) Anmeldetag: 20.07.1994
(51) Int. Cl.: C07D 487/04, C09B 57/04, C08K 5/34

(54) **Neue feinteilige cyansubstituierte Diketopyrrolopyrrolpigmente und Verwendung derselben**
In fine particles cyansubstituted diketopyrrolopyrrol pigments and their use
Dicétopyrrolopyrrol pigments cyanosubstitués en particules fines et leur utilisation

(30) Priorität: 29.07.1993 CH 229893; 18.05.1994 CH 154394
(43) Veröffentlichungstag der Anmeldung: 01.03.1995
(73) Patentinhaber: Ciba Specialty Chemicals Holding Inc., 4057 Basel (CH)
(72) Erfinder: Wallquist, Olof, Dr., CH-1723 Marly (CH)

(56) Entgegenhaltungen:
- EP-A- 0 061 426
- EP-A- 0 094 911
- EP-A- 0 181 290
- EP-A- 0 190 999
- EP-A- 0 302 018
- Pure & Appl.Chem.,Vol.58, No 6, pp.947-954, 1986
- Mol.Cryst. Liq.Cryst, Vol.96, pp.1-17 (1983)
- J.Phys.D:Appl.Phys.26 (1993) B22-B31
- Proc.Natl.Acad.Sci. USA, Vol.84, pp 4737-4738 (1987)

## Beschreibung

Die vorliegende Erfindung betrifft neue, durch bestimmte Partikelgrössenverteilung gekennzeichnete feinteilige cyansubstituierte Diketopyrrolopyrrolpigmente von ausserordentlicher Farbton-Reinheit und Transparenz, hohem Glanz, guter Dispergierbarkeit und gutem rheologischem Verhalten sowie ein Verfahren zu deren Herstellung.

1,4-Diketopyrrolo-[3,4-c]-pyrrolpigmente sind seit einigen Jahren bekannt und werden z.B. in US-Patent 4 415 685 und US-Patent 4 579 949 beschrieben. Einige davon haben sich als hochwertige Pigmente bewährt. In jüngerer Zeit ist die Nachfrage nach hochtransparenten Pigmentformen, insbesondere für die Herstellung von Metalleffektlakkierungen oder Druckfarben, erheblich gestiegen. Es stellte sich deshalb das Problem auch von bereits so hochgepriesenen Pigmenten eine reine hochtransparente Form mit guter Dispergierbarkeit und Glanzeigenschaften sowie mit gutem rheologischem Verhalten herzustellen.

Im US-Patent 4 579 949 wird die Herstellung von 1,4-Diketopyrrolo-[3,4-c]-pyrrolen durch Umsetzung eines Bernsteinsäuredialkylesters mit Nitrilen in Gegenwart einer starken Base und anschliessende Hydrolyse des entstandenen Salzes beschrieben. Es wird ausgeführt, dass die Hydrolyse in Wasser, einem Alkohol mit 1 bis 4 C-Atomen oder bevorzugt einer Säure durchgeführt werden soll und, dass transparentere Pigmentformen entstehen, wenn die Hydrolyse unter 80°C vorgenommen wird (unter Hydrolyse versteht man dabei die Ueberführung der Pigmentalkalisalze in das entsprechende Pigment, d.h. die Protonierung der Pigmentalkalisalze).

Aus US-Patent 4 659 775 sind Verfahren zur Herstellung spezieller Alkyl-diketopyrrolo-pyrrole und unsymmetrischer Diketopyrrolopyrrole ausgehend von Enamindiestern bzw. Pyrrolinonen bekannt. Aus dieser späteren Publikation geht hervor, dass die Hydrolyse bevorzugt in Wasser durchzuführen ist. Bezüglich transparente Formen, wird auch die Durchführung der Hydrolyse unter 80°C vorgeschlagen. Ähnliches wird im ebenso später erschienenen US-Patent 4 720 305 berichtet, welches die Herstellung von Diketo-pyrrolo-pyrrol-Pigmentgemischen aus Bernsteinsäurediestern und zwei verschiedenen Nitrilen betrifft. Auch gemäss dieser Publikation wird die Hydrolyse bevorzugt in Wasser durchgeführt. Zur Herstellung transparenter Formen wird hier jedoch eine nachträgliche Zerkleinerung, z.B. durch wässrige Nassmahlung, empfohlen.

US-Patent 4 931 566 beschreibt ein Verfahren zur Herstellung besonders reiner Pyrrolo-[3,4-c]-pyrrole, welches dadurch gekennzeichnet ist, dass die Hydrolyse sequentiell in mindestens zwei Schritten durchgeführt wird. Die Hydrolyse erfolgt dabei mit einer anorganischen und/oder organischen Säure, mit Wasser und Alkohol oder mit einer anorganischen oder organischen Säure, Wasser oder/und Alkohol, bevorzugt zwischen 50 und 100°C. Über Transparenz wird nichts erwähnt. Bei den spezifisch erwähnten Produkten handelt es sich durchwegs um deckende Pigmente.

Es ist nun gefunden worden, dass bei der Herstellung von cyansubstituierten Diketopyrrolopyrrolen die spezielle Kombination dreier Massnahmen bei der Herstellung und der darauffolgenden Konditionierung, nämlich
- Austragen der Pigmentsalz-Suspension auf Wasser und/oder Alkohol,
- Anwesenheit einer Säure und pH < 9 und insbesondere
- Temperaturen über 90°C
feinteilige Pigmente liefert, die dadurch gekennzeichnet sind, dass mindestens 84 Gew.% der Pigmentteilchen einen Stokes-äquivalenten Durchmesser von ≤ 0,40 µm aufweisen und sich durch überraschend hohe Farbton-Reinheit, Transparenz, Glanz, gute Dispergierbarkeit und gutes rheologisches Verhalten auszeichnen.

Die vorliegende Erfindung betrifft demnach feinteilige 1,4-Diketopyrrolo-[3,4-c]-pyrrole der Formel 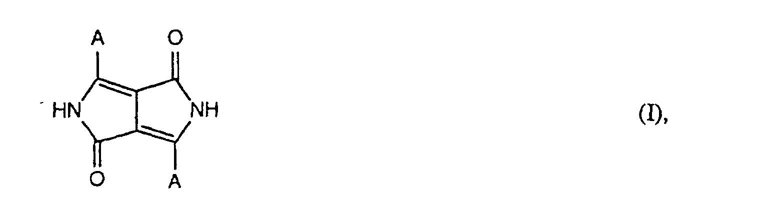 worin A einen Rest der Formel 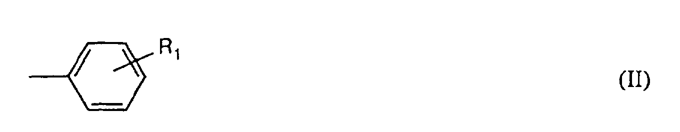 bedeutet, worin R₁ 3-CN, 4-CN oder 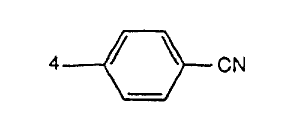 bedeutet, und die Reste A in der Formel (I) identisch sind, dadurch gekennzeichnet, dass mindestens 84 Gew.% der Pigmentteilchen einen Stokes-äquivalenten Durchmesser von ≤ 0,40 µm aufweisen.

Bevorzugt sind 1,4-Diketopyrrolo-[3,4-c]-pyrrole der Formel I, dadurch gekennzeichnet, dass mindestens 84 Gew.% der Pigmentteilchen einen Stokes-äquivalenten Durchmesser von ≤ 0,30 µm aufweisen.

Besonders bevorzugt ist das 1,4-Diketopyrrolo-[3,4-c]-pyrrol der Formel I, dadurch gekennzeichnet, dass R₁ 3-CN bedeutet und mindestens 84 Gew.% der Pigmentteilchen einen Stokes-äquivalenten Durchmesser von ≤ 0,25 µm aufweisen.

Ein weiterer Gegenstand der vorliegenden Erfindung ist das Verfahren zur Herstellung der eben beschriebenen 1,4-Diketopyrrolo-[3,4-c]-pyrrole der Formel I, durch Umsetzung von 1 Mol eines Bernsteinsäure-dicyclohexylesters, -dialkylesters, -monoalkylmonophenyl-esters oder -diphenylesters, wobei im Bernsteinsäureesterrest Alkyl C₁-C₁₈-Alkyl und Phenyl unsubstituiertes oder durch ein oder zwei Halogenatome, eine oder zwei C₁-C₆-Alkyl- oder C₁-C₆-Alkoxygruppen substituiertes Phenyl bedeuten, mit 2 Mol eines Nitrils der Formel

A-CN (III)

worin A die oben angegebene Bedeutung hat,
in einem inerten organischen Lösungsmittel in Gegenwart eines Alkalimetalls oder eines Alkali-alkoholates als starke Base bei erhöhter Temperatur zu einem Pigmentalkalimetallsalz und anschliessende Freisetzung einer Verbindung der Formel I durch Protonierung des entstandenen Pigmentalkalimetallsalzes und nachfolgende Konditionierung, dadurch gekennzeichnet, dass die Pigmentalkalisalz-Suspension in Wasser und/oder einem Alkohol ROH, worin R C₁-C₄-Alkyl ist, in Gegenwart einer Säure in genügender Menge, um den pH <9 zu halten, kontinuierlich ausgetragen wird und 30 Minuten bis 24 Stunden bei einer Temperatur über 90°C behandelt wird.

C₁-C₁₈-Alkyl steht z.B. für Methyl, Ethyl, n-Propyl, Isopropyl, n-Butyl, tert.-Butyl, Amyl, Hexyl, Decyl, Dodecyl, Tetradecyl oder Octadecyl und C₁-C₆-Alkoxy z.B. für Methoxy, Ethoxy, n-Propoxy, Isopropoxy, Butoxy oder Hexyloxy.

Die Säure kann vor oder zusammen mit der Pigmentsalz-Suspension zugegeben werden.

Es kann von Vorteil sein, ein Puffer während der Protonierung zu verwenden, wie beispielsweise ein Phosphat-, Acetat-, Citronensäure- oder Triethanolamin-Puffer.

R bedeutet als C₁-C₄-Alkyl z.B. Methyl, Ethyl, Propyl, Isopropyl, n-Butyl, tert.-Butyl. R ist bevorzugt Methyl oder Ethyl.

Bei den als Protonierungsmittel eingesetzten Säuren handelt es sich z.B. um anorganische Säuren, wie z.B. Salzsäure, Phosphorsäure und insbesondere Schwefelsäure oder um aliphatische oder aromatische Carbon- oder Sulfonsäuren, wie z.B. Ameisensäure, Essigsäure, Propionsäure, Buttersäure, Hexansäure, Oxalsäure, Benzoesäure, Phenylessigsäure, Benzolsulfonsäure oder p-Toluolsulfonsäure oder um Gemische der erwähnten Säuren. Bevorzugte organische Säuren sind Essigsäure und Ameisensäure.

Protonierung und Konditionierung erfolgen bevorzugt bei einer Temperatur zwischen 100° und 140°C während 1 bis 8 Stunden.

R₁ bedeutet vorzugsweise 3-CN.

Bei den zu verwendenden Bernsteinsäuredialkyl- oder -diphenylestern kann es sich um symmetrische oder asymmetrische Diester handeln. Bevorzugt verwendet man aber symmetrische Bernsteinsäurediester, insbesondere symmetrische Bernsteinsäuredialkylester. Liegt ein Bernsteinsäurediphenyl-oder-monophenylmonoalkylestervor, so kann Phenyl beispielsweise unsubstituiertes oder durch ein oder zwei Halogenatome, wie Chlor, C₁-C₆-Alkylgruppen, wie Methyl, Ethyl, Isopropyl odertert.-Butyl, oder C₁-C₆-Alkoxygruppen, wie Methoxy oder Ethoxy, substituiertes Phenyl bedeuten. Phenyl bedeutet bevorzugt unsubstituiertes Phenyl. Handelt es sich um einen Bemsteinsäuredialkyl- oder -monoalkylmonophenylester, so kann Alkyl unverzweigt oder verzweigt sein, bevorzugt verzweigt, und vorzugsweise 1 bis 12, insbesondere 1 bis 8 und besonders bevorzugt 1 bis 5 C-Atome enthalten. Verzweigtes Alkyl ist bevorzugt sek.- oder tert.-Alkyl, wie z.B. Isopropyl, sek.-Butyl, tert.-Butyl und tert.-Amyl. Ganz bevorzugt verwendet man symmetrische verzweigte Bernsteinsäuredialkylester, worin jeder Alkylrest im Bernsteinsäureesterrest 3 bis 5 C-Atome aufweist.

Beispiele für Bernsteinsäurediester sind Bernsteinsäuredimethylester, -diethylester, -dipro-pylester, -dibutylester, -dipentylester, -dihexylester, -diheptylester, -dioctylester, -diiso-propylester, -di-sec.-butylester, -di-tert.-butylester, -di-tert.-amylester, -di-[1,1-dimethyl-butyl]-ester, -di-[1,1,3,3-tetramethylbutyl]-ester, -di-[1,1-dimethylpentyl]-ester, -di-[1-methyl-1-ethyl-butyl]-ester, -di-[1,1-diethylpropyl]-ester, -diphenylester, -di-[4-methyl-phenyl]-ester, -di-[2-methylphenyl]-ester, -di-[4-chlorphenyl]-ester, -di-[2,4-dichlor-phenyl]-ester und -monoethyl-monophenylester.

Die oben aufgeführten Bernsteinsäurediester und die Nitrile der Formel III sind bekannte Verbindungen und können nach bekannten Verfahren hergestellt werden.

Man führt die Umsetzung des Bernsteinsäurediesters mit dem Nitril der Formel III in einem organischen Lösungsmittel durch. Als Lösungsmittel eignen sich beispielsweise primäre, sekundäre oder tertiäre Alkohole mit 1 bis 10 C-Atomen, wie Methanol, Ethanol, n-Propanol, Isopropanol, n-Butanol, sek.-Butanol, tert.-Butanol, n-Pentanol, 2-Methyl-2-butanol, 2-Methyl-2-pentanol, 3-Methyl-3-pentanol, 2-Methyl-2-hexanol, 3-Ethyl-3-pentanol und 2,4,4-Trimethyl-2-pentanol, Glykole, wie Ethylenglykol oder Diethylenglykol, ferner Ether, wie Tetrahydrofuran oder Dioxan, oder Glykolether, wie Ethylen-glykol-mono- oder -di-methylether, Ethylenglykol-mono- oder -di-ethylether, Diethylenglykol-monomethylether oder Diethylenglykolmonoethylether, ferner dipolar-aprotische Lösungsmittel, wie Acetonitril, Benzonitril, Dimethylformamid, N,N-Dimethylacetamid, Nitrobenzol und N-Methylpyrrolidon, aliphatische oder aromatische Kohlenwasserstoffe, wie Benzol oder durch Alkyl, Alkoxy oder Halogen substituiertes Benzol, wie Toluol, Xylole, Anisol oder Chlorbenzol, oder aromatische N-Heterocyclen, wie Pyridin, Picolin oder Chinolin. Die oben genannten Lösungsmittel können auch als Mischungen eingesetzt werden. Zweckmässigerweise verwendet man 5-20 Gew.-Teile Lösungsmittel auf 1 Gew.-Teil der Reaktionsteilnehmer.

Im erfindungsgemässen Verfahren verwendet man als Lösungsmittel bevorzugt einen Alkohol, insbesondere einen sekundären oder tertiären Alkohol. Bevorzugte tertiäre Alkohole sind tert.-Butanol und tert.-Amylalkohol. Dabei sind auch Gemische davon, oder Gemische dieser bevorzugten Lösungsmittel mit aromatischen Kohlenwasserstoffen, wie Toluol oder Xylole, oder mit durch Halogen substituierten Benzolen, wie Chlorbenzol oder o-Dichlorbenzol, von grossem Interesse.

Anmeldungsgemäss geeignete starke Basen sind Alkalimetalle, wie Lithium, Natrium und Kalium, und Alkalialkoholate, die sich insbesondere von primären, sekundären oder tertiären aliphatischen Alkoholen mit 1 bis 10 C-Atomen ableiten, wie z.B. Lithium-, Natrium- oder Kaliummethylat, -ethylat, -n-propylat, -isopropylat, -n-butylat, -sek.butylat, -tert.- butylat, -2-methyl-2-butylat, -2-methyl-2-pentylat, -3-methyl-3-pentylat und -3-ethyl-3-pentylat. Man kann aber auch ein Gemisch der oben genannten Alkalialkoholate verwenden. Bevorzugt verwendet man Alkalialkoholate, wobei Alkali insbesondere Natrium oder Kalium bedeutet, und das Alkoholat sich bevorzugt von einem sekundären oder tertiären Alkohol ableitet. Besonders bevorzugte starke Basen sind daher z.B. Natrium- oder Kaliumisopropylat, -sek.-butylat, -tert.-butylat und -tert-amylat. Dabei können die Alkalialkoholate auch in situ hergestellt werden, indem man den entsprechenden Alkohol mit dem Alkalimetall umsetzt.

Im erfindungsgemässen Verfahren kann man die starke Base beispielsweise in einer Menge von 0,1 bis 10 Mol, bevorzugt 1,9 bis 4,0 Mol, bezogen auf 1 Mol des Bemsteinsäurediesters, einsetzen. Obwohl grundsätzlich stöchiometrische Mengen an Base genügen, beeinflusst in vielen Fällen ein Basenüberschuss die Ausbeute günstig.

Die Umsetzung kann beispielsweise bei einer Temperatur von 60 bis 140°C, vorzugsweise aber von 80 bis 120°C, durchgeführt werden.

Zur Umsetzung des Bemsteinsäurediesters mit dem Nitril der Formel III ist es grundsätzlich möglich, bei tieferer Temperatur alle Komponenten vorzulegen und dann das Gemisch in den Bereich der Reaktionstemperatur zu erwärmen, oder in beliebiger Reihenfolge die einzelnen Komponenten im Bereich der Reaktionstemperatur zuzugeben. Eine bevorzugte Ausführungsform, die sich in der Regel besonders günstig auf die Ausbeute auswirkt, besteht darin, dass man das umzusetzende Nitril zusammen mit der starken Base vorlegt und den Bernsteinsäurediester im Bereiche der Reaktionstemperatur zudosiert. Eine weitere Möglichkeit besteht darin, dass man den Bernsteinsäurediester und das umzusetzende Nitril gleichzeitig zur vorgelegten Base zudosiert. Es ist durchaus möglich, das erfindungsgemässe Verfahren nicht nur batchweise, sondern auch kontinuierlich durchzuführen.

Insbesondere bei Bernsteinsäurediestern mit niederen Alkylresten und bei Alkoholaten, die sich aus niederen Alkoholen ableiten, wie z.B. Methanol, Ethanol, n-Propanol, Isopropanol oder tert.-Butanol, kann es sich als vorteilhaft erweisen, den bei der Umsetzung entstehenden niederen Alkohol laufend aus dem Reaktionsmedium zu entfernen, um höhere Ausbeuten zu erzielen.

Verwendet man als Lösungsmittel einen Alkohol und als Base ein Alkoholat, so kann es vorteilhaft sein, einen Alkohol und ein Alkoholat mit gleichen Alkylteilen zu wählen. Ebenso vorteilhaft kann es sein, wenn zudem noch der Bernsteinsäurediester ebensolche Alkylgruppen enthält.

Zur Protonierung des erhaltenen Pigmentsalzes kann man entweder das Pigmentalkalisalz zum erfindungsgemässen, aus dem Wasser und/oder Alkohol und der Säure bestehenden Protonierungsmittel oder das Pigmentalkalisalz und die Säure gleichzeitig zum Wasser und/oder Alkohol zugeben. Das Wasser und/oder der Alkohol kann in beliebigen Mischungsverhältnissen von 5 bis 20 Gew.Teilen auf 1 Teil des entstandenen Pigmentalkalisalzes eingesetzt werden. Die Säure wird zweckmässig in einer Menge von 0,5 bis 3 Aequivalenten, bezogen auf die Base eingesetzt, allenfalls in genügender Menge, um den pH <9 am Ende der Protonierung zu erreichen.

Die Verbindungen der Formel l können als Pigmente für hochmolekulare organische Materialien verwendet werden. Dabei lassen sich die Pigmente meistens direkt in der Pigmentform einsetzen, wie sie nach dem erfindungsgemässen Verfahren anfallen. Ihre Kristallmorphologie kann je nach Verwendungszweck, und sofern notwendig, durch eine der zahlreichen üblichen nachträglichen Behandlungen noch optimiert werden.

Je nach Verwendungszweck kann es vorteilhaft sein, Gemische von Verbindungen der Formel herzustellen. Dies lässt sich beispielsweise dadurch erreichen, dass man unabhängig voneinander hergestellte, verschiedene Reaktionslösungen vor der Protonierung mischt, sie zusammen protoniert und dann das erhaltene Produkt isoliert.

Hochmolekulare organische Materialien, die mit den Verbindungen der Formel I gefärbt bzw. pigmentiert werden können, sind z.B. Celluloseether und -ester, wie Ethylcellulose, Nitrocellulose, Celluloseacetat und Cellulosebutyrat, natürliche Harze und Kunstharze, wie Polymerisationsharze oder Kondensationsharze, z.B. Aminoplaste, insbesondere Harnstoff- und Melamin-Formaldehydharze, Alkydharze, Phenoplaste, Polycarbonate, Polyolefine, wie Polyethylen und Polypropylen, Polystyrol, Polyvinylchlorid, Polyacrylnitril, Polyacrylsäureester, Polyamide, Polyurethane, Polyester, Gummi, Casein, Silikon und Silikonharze, einzeln oder in Mischungen.

Dabei spielt es keine Rolle, ob die erwähnten hochmolekularen organischen Verbindungen als plastische Massen, Schmelzen oder in Form von Spinnlösungen, Lacken, Anstrichstoffen oder Druckfarben vorliegen. Je nach Verwendungszweck erweist es sich als vorteilhaft, die Verbindungen der Formel l als Toner oder in Form von Präparaten einzusetzen. Bezogen auf das zu pigmentierende hochmolekulare organische Material kann man die Verbindungen der Formel 1 in einer Menge von 0,01 bis 30 Gew.-%, vorzugsweise 0,1 bis 10 Gew.-%, einsetzen.

Je nach Konditionierverfahren oder Applikationszweck kann es von Vorteil sein, dem Pigment gewisse Mengen an texturverbessernden Mitteln vor oder nach dem Konditionierprozess zuzufügen, sofern diese keine negative Wirkung bei der Verwendung der erfindungsgemässen Pigmentzusammensetzungen (insbesondere in Polyethylen) haben. Als solche kommen insbesondere Fettsäuren mit mindestens 18 C-Atomen, beispielsweise Stearin- oder Behensäure oder deren Amide oder Metallsalze, insbesondere Mg-Salze, sowie Weichmacher, Wachse, Harzsäuren, wie Abietinsäure, Kolophoniumseife, Alkylphenole oder aliphatische Alkohole, wie Stearylalkohol oder aliphatische 1,2-Dihydroxyverbindungen mit 8 bis 22 C-Atomen, wie 1,2-Dodecandiol, ferner modifizierte Kolophoniummaleinatharze oder Fumarsäurekolophoniumharze in Betracht. Die texturverbessernden Mittel werden vorzugsweise in Mengen von 1,0-50 Gew.%, insbesondere 5-40 Gew.%, bezogen auf das Endprodukt, zugesetzt. Die obenerwähnten 1,2-Dihydroxyverbindungen, insbesondere 1,2-Dodecandiol, dienen auch zur Verbesserung der Filtration der suspendierten Pigmentzusammensetzung.

Die erhaltenen Ausfärbungen, beispielsweise in Kunststoffen, Fasern, Lacken oder Drucken, zeichnen sich durch eine hohe Farbtonreinheit, hohe Farbstärke, hohe Transparenz, gute Ueberlackier-, Migrations-, Hitze-, Lichtund Wetterbeständigkeit, sowie durch einen guten Glanz, aus.

Die erfindungsgemässen Verbindungen der Formel I zeichnen sich aber, wie bereits erwähnt, ganz besonders durch eine hervorragende Farbton-Reinheit, hohe Transparenz, gute Dispergierbarkeit, gutes rheologisches Verhalten und durch einen hohen Glanz der damit erhaltenen Ausfärbungen aus. Demnach eignen sie sich vorzugsweise zum Färben von Kunststoffen, wässrigen und/oder lösungsmittelhaltigen Lacken, insbesondere Automobillacken oder Druckfarben. Ganz besonders bevorzugt ist ihre Verwendung in Druckfarben.

Die nachfolgenden Beispiele dienen zur Erläuterung der Erfindung. Sofern nichts anderes vermerkt, bedeuten Prozente Gewichtsprozente.

Beispiel 1: In einem Sulfierkolben werden unter Stickstoff 1150 ml tert.-Amylalkohol vorgelegt. Nach der Zugabe von 38,4 g Natrium wird das Gemisch auf 95-102°C aufgewärmt. Das geschmolzene Natrium wird nun über Nacht bei 100-105°C kräftig gerührt. Nach Abkühlen der Lösung auf 85°C werden 145,6 g 3-Cyanbenzonitril zugegeben, mit 30 ml tert.-Amylalkohol nachgespült und anschliessend während 2 Stunden bei 80-85°C 127,8 g Bernsteinsäure-diisopropylester zugetropft. Dann wird das Reaktionsgemisch noch 4 Stunden bei dieser Temperatur gerührt und danach auf ein Gemisch von 462 ml Wasser, 1738 ml Methanol und 190,3 g konzentrierter Salzsäure ausgetragen, bei 130°C während 6 Stunden gerührt (pH < 7), abgekühlt, filtriert, mit Methanol/ Wasser und nochmals mit Wasser gewaschen. Das erhaltene Pigment wird bei 80°C im Vakuumschrank getrocknet. Man erhält 168,5 g eines orangen Pulvers, das in PVC eingearbeitet eine reine, transparente, orange Ausfärbung ergibt.

| Analyse | C | H | N |
|---|---|---|---|
| Berechnet | 71,00 % | 2,98 % | 16,56 % |
| Gefunden | 70,01 % | 3,08 % | 16,42 % |

Beispiel 2: Man verfährt wie in Beispiel 1, ersetzt jedoch das Methanol/Wasser/Salzsäure-Gemisch mit 2560 ml Wasser und 83,0 g 96 %ige Schwefelsäure und führt nach 6-stündigem Rühren bei 140°C eine Wasserdampfdestillation durch. Nach dem Abfiltrieren, Waschen mit Wasser und Trocknen erhält man 178,9 g eines orangen Pulvers, das in PVC eine reine, transparente orange Ausfärbung ergibt.

| Analyse | C | H | N |
|---|---|---|---|
| Berechnet | 71,00 % | 2,98 % | 16,56 % |
| Gefundenz | 70,50 % | 3,15 % | 16,16 % |

Beispiel 3: In einem Sulfierkolben werden unter Stickstoff 700 ml tert.-Amylalkohol vorgelegt und auf 80°C aufgeheizt. Nach der Zugabe von 24,2 g Natrium wird das Gemisch auf etwa 95-110°C erhitzt. Das geschmolzene Natrium wird über Nacht bei 100-110°C kräftig gerührt. Nach dem Abkühlen auf 80°C werden 89,7 g 3-Cyanbenzonitril zugegeben, dann innerhalb 2 Stunden bei 80°C 80,9 g Bernsteinsäurediisopropylester zugetropft und über Nacht bei der gleichen Temperatur gerührt. Das Reaktionsgemisch wird danach gleichzeitig mit 60 %iger Schwefelsäure innerhalb von etwa 3 Stunden einem Gemisch aus 600 ml Wasser, 600 ml Methanol und 8,2 g Natriumdihydrogenphosphat unter Rückfluss zugeführt. Es wird so viel Schwefelsäure zugetropft, dass der pH zwischen 6 und 8 gehalten wird. Das Gemisch wird 4 Stunden unter Rückfluss und anschliessend 6 Stunden bei 100°C gerührt, dann abgekühlt, abfiltriert, der Rückstand wird mit Methanol/Wasser und danach mit Wasser gewaschen und bei 80°C im Vakuumtrockenschrank getrocknet. Man erhält 104,4 g eines orangen Pulvers, das in PVC einen reinen, transparenten, orangen Farbton ergibt.

| Analyse | C | H | N |
|---|---|---|---|
| Berechnet | 71,00 % | 2,98 % | 16,56 % |
| Gefunden | 68,80 % | 3,07 % | 16,01 % |

Beispiel 4: In einem Sulfierkolben werden unter Stickstoff 160 ml tert.-Amylalkohol vorgelegt. Nach der Zugabe von 6,9 g Natrium wird das Gemisch auf etwa 95-108°C erhitzt und das geschmolzene Natrium wird über Nacht bei 100-105°C kräftig gerührt. Nach dem Abkühlen auf 80°C werden 25,6 g 4-Cyanbenzonitril zugegeben, dann innerhalb von 2 Stunden bei der gleichen Temperatur 22,2 g Bernsteinsäurediisopropylester zugetropft und danach 22 Stunden bei 80°C gerührt. Das Reaktionsgemisch wird anschliessend auf ein Gemisch aus 30,5 ml 36 %iger Salzsäure, 345 ml Methanol und 115 ml Wasser ausgetragen und 6 Stunden bei 130°C gerührt. Nach Filtration wird der Rückstand mit Methanol und Wasser gewaschen und im Vakuumtrockenschrank bei 80°C getrocknet. Man erhält 32,4 g eines gut dispergierbaren dunklen Pulvers, das in PVC eine dunkelrote transparente Ausfärbung ergibt.

| Analyse | C | H | N |
|---|---|---|---|
| Berechnet | 71,00 % | 2,98 % | 16,58 % |
| Gefunden | 69,59 % | 3,05 % | 16,20 % |

Beispiel 5: 5 g des Pigments aus Beispiel 1, 56,56 g Alkydharz® ALKYDAL F310 (Bayer), 60 %ig in Xylol, 21,70 g Xylol, 0,94 g Silikonöl (1 %ig in Xylol) und 13,55 g Melaminharz® CYMEL 327 (Cyanamid). 90 % in Isobutanol werden zusammen in einer Schüttelmaschine (® Skandex-Disperser BA-520, in Anlehnung an DIN 53238, Teil 10) bis Stufe 6 (DIN 53238, Teil 24) erreicht wird, dispergiert. Der so erhaltene Volltonlack kann nach Verdünnung für die Bestimmung der Teilchengrössenverteilung durch Photosedimentometrie (vgl. Herbst & Hunger, Industrielle organische Pigmente, VCH 1987, S. 32-34 und 40-43 und K. Brugger, Powder Technology 13, 215-221 (1976)) verwendet werden. Im vorliegenden Fall weisen mindestens 84 Gew.% der Pigmentteilchen einen Stokes-äquivalenten Durchmesser von ≤ 0,25 µm auf.
Der erhaltene Volltonlack kann auch mit einem Spiralrakel (100 µm Nassfilm) auf eine Polyethylen-Transparentfolie appliziert werden. Der Lack wird dann 15 Minuten bei Raumtemperatur abdunsten gelassen und anschliessend 30 Minuten bei 115°C eingebrannt.

Beispiel 6: 7,5 g des Pigments aus Beispiel 1, 98,9 g CAB-Lösung bestehend aus

| | |
|---|---|
| 41,0 g | Celluloseacetobutyrat® CAB 531.1, 20 %ig in Butanol/Xylol 2:1 (Eastman Chem.) |
| 1,5 g | Zirkonium Octoat, |
| 18,5 g | ® SOLVESSO 150* (ESSO), |
| 21,5 g | Butylacetat und |
| 17,5 g | Xylol, |

| | |
|---|---|
| * Aromatische Kohlenwasserstoffe | |

36,5 g Polyesterharz ® DYNAPOL H700 (Dynamit Nobel), 4,6 g Melaminharz MAPRENAL MF650 (Hoechst) und 2,5 g Dispergiermitel ® DISPERBYK 160 (Byk Chemie) werden zusammen während 90 Minuten mit einer Schüttelmaschine dispergiert (Total Lack 150 g; 5 % Pigment).

27,69 g des so erhaltenen Volltonlacks werden für die Base-coat-Lackierung mit 17,31 g Al-Stammlösung (8 %ig) bestehend aus

| | |
|---|---|
| 12,65 g | ® SILBERLINE SS 3334AR, 60 %ig (Silberline Ltd.) |
| 56,33 g | CAB Lösung (Zusammensetzung wie oben) |
| 20,81 g | Polyesterharz ® DYNAPOL H700 |
| 2,60 g | Melaminharz ® MAPRENAL MF650 |
| 7,59 g | ® SOLVESSO 150 |

gemischt und auf ein Aluminiumblech spritzappliziert (Nassfilm ca. 20 µm). Nach einer Abdunstzeit von 30 Minuten bei Raumtemperatur wird ein TSA-Lack bestehend aus

| | |
|---|---|
| 29,60 g | Acrylharz ® URACRON 2263 XB, 50 %ig in Xylol/Butanol (Chem. Fabrik Schweizerhalle), |
| 5,80 g | Melaminharz ® CYMEL 327, 90 %ig in Isobutanol, |
| 2,75 g | Butylglycolacetat, |
| 5,70 g | Xylol, |
| 1,65 g | n-Butanol |
| 0,50 g | Siliconöl, 1 %ig in Xylol, |
| 3,00 g | Lichtschutzmittel ® TINUVIN 900, 10 %ig in Xylol (Ciba) |
| 1,00 g | Lichtschutzmittel ®TINUVIN 292, 10 %ig in Xylol (Ciba) |

als Top-coat-Lackierung spritzappliziert (Nassfilm ca. 50 µm). Anschliessend wir der Lack nach weiteren 30 Minuten Abdunsten bei Raumtemperatur, 30 Minuten bei 130°C eingebrannt.

### Beispiel 7: Herstellung einer Tief-/Flexodruckfarbe.

| | | |
|---|---|---|
| 15 g | des Pigments aus Beispiel 1, | |
| 20 g | Klarlacke bestehend aus | |
| | 20 g | Nitrocellulose A Typ |
| | 4 g | Dioctylphthalat |
| | 56 g | Ethanol und |
| | 20 g | Ethylacetat |
| | und | |
| 25 g | Ethanol | |

werden mittels Hochgeschwindigkeitsrührer (Dissolver bei 15 m/s) 30 Minuten dispergiert.
Anschliessend wird der Ansatz mit 40 g des oben bereits beschriebenen Klarlacks ergänzt und weitere 5 Minuten mit dem Dissolver dispergiert. Dieser Mahlansatz wird mittels einer Pumpe mit Grobfiltrierung in eine Perlmühle eingeleitet und darin feindispergiert. Mit dieser Druckfarbe erhält man sowohl im Tief-/Flexodruck, wie auch im Offsetdruck aussergewöhnliche Transparenz/Glanz-Eigenschaften.

## Patentansprüche

1. Feinteilige 1,4-Diketopyrrolo-[3,4-c]-pyrrole der Formel 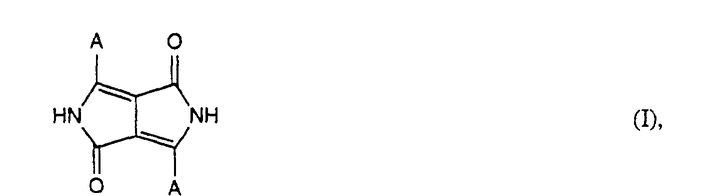 worin A einen Rest der Formel 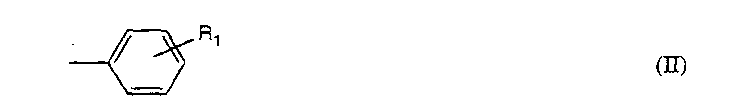 bedeutet, worin R₁ 3-CN, 4-CN oder 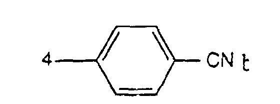 bedeutet,
und die Reste A in der Formel (I) identisch sind,
**dadurch gekennzeichnet, dass** mindestens 84 Gew.% der Pigmentteilchen einen Stokes-äquivalenten Durchmesser von ≤ 0,40 µm aufweisen.

2. 1,4-Diketopyrrolo-[3,4-c]-pyrrole der Formel l, gemäss Anspruch 1, **dadurch gekennzeichnet, dass** mindestens 84 Gew.% der Pigmentteilchen einen Stokes-äquivalenten Durchmesser von ≤ 0,30 µm aufweisen.

3. 1,4-Diketopyrrolo-[3,4-c]-pyrrol der Formel l, gemäss Anspruch 1, **dadurch gekennzeichnet, dass** R₁ 3-CN bedeutet und mindestens 84 Gew.% der Pigmentteilchen einen Stokes-äquivalenten Durchmesser von ≤ 0,25 µm aufweisen.

4. Verwendung der 1,4-Diketopyrrolo-[3,4-c]-pyrrole gemäss Anspruch 1 als Pigmente zum Einfärben von hochmolekularem organischem Material.

## Claims

1. A finely particulate 1,4-diketopyrrolo[3,4-c]pyrrole of formula 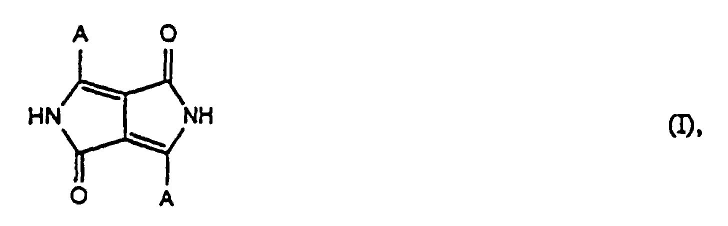 in which A is a radical of formula 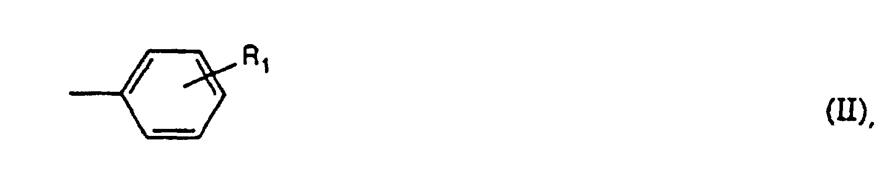 in which R₁ is 3-CN, 4-CN or 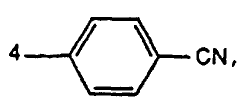 and the radicals A in formula (I) are identical, wherein at least 84 % of the pigment particles have a Stokes equivalent diameter of ≤ 0.40 µm.

2. A 1,4-diketopyrrolo[3,4-c]pyrrole of formula I according to claim 1, wherein at least 84 % of the pigment particles have a Stokes equivalent diameter of ≤ 0.30 µm.

3. A 1,4-diketopyrrolo[3,4-c]pyrrole of formula I according to claim 1, wherein R₁ is 3-CN and at least 84 % of the pigment particles have a Stokes equivalent diameter of ≤ 0.25 µm.

4. Use of a 1,4-diketopyrrolo[3,4-c]pyrrole according to claim 1 as pigment for colouring organic material of high molecular weight.

## Revendications

1. 1,4-Dicétopyrrolo-[3,4-c]-pyrroles finement divisés de formule 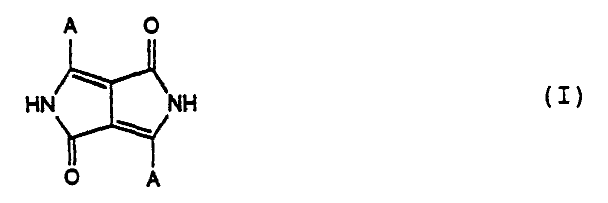 où
A représente un reste de formule 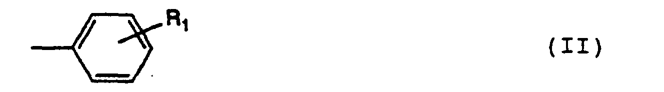 où R₁ représente des groupes 3-CN, 4-CN ou 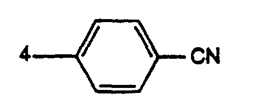 et les restes A à la formule (I) sont identiques,
**caractérisé en ce que** au moins 84 % des particules pigmentaires présentent un diamètre équivalent de Stockes ≤0, 40 µm.

2. 1,4-Dicétopyrrolo-[3,4-c]-pyrroles de formule I selon la revendication 1, **caractérisés en ce que** au moins 84 % des particules pigmentaires présentent un diamètre équivalent de Stockes ≤0,30 µm.

3. 1,4-Dicétopyrrolo-[3,4-c]-pyrroles de formule I selon la revendication 1, **caractérisés en ce que** R₁ représente un groupe 3-CN et au moins 84 % des particules pigmentaires présentent un diamètre équivalent de Stockes ≤ 0,25 µm.

4. Utilisation des 1,4-dicétopyrrolo-[3,4-c]-pyrroles selon la revendication 1 en tant que pigment pour la coloration de matières organiques de haut poids moléculaire.
